# DEMANDE DE BREVET EUROPEEN

(11) **EP 1 702 609 A1**
(43) Date de publication de la demande: **20.09.2006**
(21) Numéro de dépôt: 06110082.2
(22) Date de dépôt: 17.02.2006
(51) Int. Cl.: A61K 8/42, A61K 8/60, A61Q 5/02, A61Q 5/12, A61Q 19/10

(54) **Composition cosmétique de nettoyage contenant un composé d'urée et un polymère**

(30) Priorité: 17.03.2005 FR 0550683
(71) Demandeur: L'ORÉAL, 75008 Paris (FR)
(72) Inventeur: Fonolla Moreno, Angeles, 75013, Paris (FR); Piot, Bertrand, 75009, Paris (FR)
(74) Mandataire: Rasson, Catherine

(57) **Abrégé**

L'invention se rapporte à une composition cosmétique de nettoyage contenant dans un milieu aqueux, (1) au moins un tensioactif non ionique et (2) au moins un composé de formule (I) suivante : 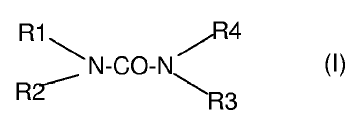 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle, ainsi que leurs sels, leurs solvats, et leurs isomères, et (3) au moins un sel d'ammonium quaternaire polymérique.

Le composé de formule (I) est de préférence l'hydroxyéthylurée.

Le tensioactif non ionique peut être choisi notamment parmi les alkylpolyglucosides, les esters de glycérol oxyalkylénés, les esters de sucre oxyalkylénés, et leurs mélanges.

L'invention se rapporte aussi à l'utilisation de la dite composition comme produit de nettoyage et/ou de démaquillage de la peau et/ou des lèvres, ou comme produit de nettoyage des cheveux.

## Description

L'invention se rapporte à une composition cosmétique de nettoyage contenant dans un milieu aqueux, un composé d'urée hydroxylé, un tensioactif moussant non ionique et un sel d'ammonium quaternaire polymérique, et à ses utilisations pour le nettoyage et/ou le démaquillage de la peau humaine, des lèvres, et/ou des cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage s'accumulent dans les replis cutanés et peuvent obstruer les pores de la peau et entraîner l'apparition de boutons. Un moyen pour bien nettoyer la peau est d'utiliser des produits de nettoyage moussants.

Une des difficultés majeures de la formulation de produits moussants consiste à mettre au point des produits ayant un très bon pouvoir moussant pour assurer un bon nettoyage et pouvant se rincer facilement, tout en respectant la barrière cutanée, c'est-à-dire sans dessécher la peau.

Pour la formulation d'un produit d'une telle qualité, il est nécessaire d'utiliser un fort taux de tensioactifs moussants pour assurer une mousse rapide, abondante, dense, facile à étaler et qui permettra un bon rinçage, et d'ajouter un agent hydratant qui s'avère indispensable pour avoir une bonne tolérance et pour assurer le respect du film protecteur hydrolipidique et éviter ainsi le dessèchement de la peau.

Or, il a déjà été constaté que la plupart des compositions ayant de bonnes propriétés moussantes contiennent des tensioactifs très desséchants pour la peau et que l'ajout de la glycérine qui est un actif hydratant classiquement connu, permet d'atténuer cet effet de dessèchement mais présente l'inconvénient de diminuer le pouvoir moussant de la composition (mauvais démarrage de mousse) et de rendre le rinçage plus difficile. En outre, pour avoir un effet hydratant, il faut une certaine quantité de glycérine, et la composition moussante obtenue a un toucher lourd et collant lors de l'application sur la peau.

Il subsiste donc le besoin de disposer de compositions cosmétiques de nettoyage, qui ne soient pas desséchantes pour la peau et soient donc hydratantes tout en ayant néanmoins un bon pouvoir moussant (bon démarrage de mousse) et une bonne rincabilité, ainsi qu'un toucher léger et non collant à l'application sur la peau.

La demanderesse a découvert de manière surprenante que les compositions de nettoyage contenant l'association de tensioactif moussant non ionique, sel d'ammonium quaternaire polymérique et composé d'urée hydroxylé permettaient de résoudre le problème à la base de l'invention.

Ainsi, l'invention a pour objet une composition cosmétique de nettoyage contenant dans un milieu aqueux, (1) au moins un tensioactif moussant non ionique et (2) au moins un composé de formule (I) suivante : 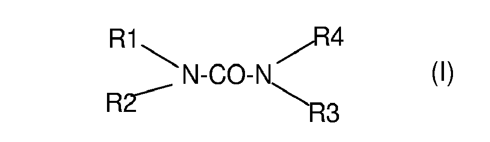 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle, ainsi que leurs sels, leurs solvats, et leurs isomères, et (3) au moins un sel d'ammonium quaternaire polymérique.

Certes, il est connu par le document DE2703185 d'introduire des dérivés d'urée de formule (I) dans des compositions nettoyantes, mais rien ne laissait présager qu'en associant ce dérivé avec des tensioactifs non ioniques et un sel d'ammonium quaternaire, on obtiendrait une composition ayant non seulement un bon pouvoir hydratant, mais aussi un toucher léger et non collant, et ayant de plus un bon démarrage de mousse, même en présence d'une quantité importante de composé d'urée.

Ainsi, la composition de l'invention présente l'avantage de ne pas dessécher la peau tout en ayant un bon pouvoir moussant et tout en étant bien rinçable. La mousse obtenue est abondante, dense et crémeuse et se rince très bien.

On entend ici par « milieu aqueux », un milieu contenant de l'eau et éventuellement un ou plusieurs solvants organiques hydrosolubles. Dans ce milieu aqueux, la quantité d'eau est de préférence d'au moins 20 % en poids; elle va de préférence de 20 à 95 % en poids, mieux de 30 à 90 % en poids et encore mieux de 40 à 85 % en poids par rapport au poids total de la composition.

En outre, la composition de l'invention étant une composition cosmétique, elle contient un milieu physiologiquement acceptable. On entend ici par « milieu physiologiquement acceptable », un milieu compatible avec la peau, les muqueuses, les yeux. Il s'agit de préférence d'un milieu cosmétiquement acceptable, c'est-à-dire qui, en outre, présente une couleur, une odeur et un toucher agréables et qui ne génère pas d'inconforts inacceptables (picotements, tiraillements, rougeurs), susceptibles de détourner la consommatrice d'utiliser cette composition.

De façon avantageuse, le pH du milieu aqueux est compatible avec les matières kératiniques et notamment avec la peau. Ce pH va de préférence de 3 à 8,5, mieux de 3,5 à 8, préférentiellement de 5 à 7,5.

La composition de nettoyage de l'invention est une composition moussante qui est rincée après application sur la peau.

### Composé d'urée hydroxylé

Le composé d'urée présent dans la composition selon l'invention est un composé de formule (I) suivante : 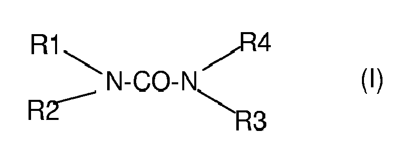 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle, ainsi que leurs sels, leurs solvats, et leurs isomères.

Dans les composés de formule (I) :
- De préférence R1 désigne un groupe hydroxyalkyle en C2-C6 et R2, R3, R4 désignent, indépendamment l'un de l'autre, un atome d'hydrogène ou un groupe alkyle en C1-C4 ;
- Préférentiellement R1 désigne un groupe hydroxyalkyle en C2-C6 comprenant de 1 à 5 groupes hydroxyles, notamment 1 groupe hydroxyle, et R2, R3, R4 désignent un atome d'hydrogène ;
- Plus préférentiellement, R1 désigne un groupe hydroxyalkyle en C2-C4 comprenant 1 groupe hydroxyle et R2, R3, R4 désignent un atome d'hydrogène.

Comme groupes alkyle en C1-C4, on peut citer notamment les groupes méthyle, éthyle, n-propyle, isopropyle, n-butyle, iso-butyle et tert-butyle.

Parmi les groupes hydroxyalkyle en C1-C6, on préfère ceux contenant un seul groupe hydroxyle et en particulier les groupes hydroxyéthyle, hydroxypropyle, hydroxybutyle, hydroxypentyle et hydroxyhexyle.

Comme sels de tels composés, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, acide sulfonique ou acide phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Comme composés de formule (I) préférés, on peut citer le N-(2-hydroxyéthyl)-urée ; le N-(2-hydroxypropyl)-urée ; le N-(3-hydroxypropyl)-urée ; le N-(2,3-dihydroxypropyl)-urée ; le N-(2,3,4,5,6-pentahydroxyhexyl)-urée ; le N-méthyl-N-(1,3,4,5,6-pentahydroxy-2-hexyl)-urée ; le N-méthyl-N'-(1-hydroxy-2-méthyl-2-propyl)-urée ; le N-(1-hydroxy-2-méthyl-2-propyl)-urée ; le N-(1,3-dihydroxy-2-propyl)-urée ; le N-(tris-hydroxyméthyl-méthyl)-urée ; le N-éthyl-N'-(2-hydroxyéthyl)-urée ; le N,N-bis-(2-hydroxyéthyl)-urée ; le N,N'-bis-(2-hydroxyéthyl)-urée ; le N,N-bis-(2-hydroxypropyl)-urée ; le N,N'-Bis-(2-hydroxypropyl)-urée ; le N,N-Bis-(2-hydroxyéthyl)-N'-propyl-urée ; le N,N-Bis-(2-hydroxypropyl)-N'-(2-hydroxyéthyl)-urée ; le N-tert.Butyl-N'-(2-(hydroxyéthyl)-N'-(2-(hydroxypropyl)-urée; le N-(1,3-dihydroxy-2-propyl)-N'-(2-hydroxyéthyl)-urée ; le N,N-Bis-(2-hydroxyéthyl)-N',N'-dimethyl-urée ; le N,N,N',N'-tetrakis-(2-hydroxyéthyl)-urée ; le N',N'-Bis-(2-hydroxyéthyl)-N',N'-bis-(2-hydroxypropyl)-urée ; et leurs mélanges.

De préférence, le composé de formule (I) est le N-(2-hydroxyéthyl)-urée.

Les composés de formule (I) sont des composés connus et notamment décrits dans la demande DE-A-2703185. Parmi ceux-ci, le N-(2-hydroxyéthyl)-urée est en outre disponible dans le commerce, sous forme de mélange à 50% en poids dans l'eau, auprès de la société National Starch sous la dénomination commerciale Hydrovance® .

Le composé de formule (I) peut être présent dans la composition selon l'invention en une quantité (en matière active) allant de 0,5 % à 30 % en poids, par rapport au poids total de la composition, de préférence allant de 1 % à 20 % en poids, et préférentiellement allant de 2 % à 10 % en poids.

### Tensioactif moussant non ionique

La composition contient un ou plusieurs tensioactifs moussants non ioniques. Ces tensioactifs sont des tensioactifs moussants aptes à nettoyer la peau.

Les tensioactifs moussants sont des détergents et se différencient des émulsionnants par la valeur de leur HLB (Hydrophilic Lipophilic balance), le HLB étant le rapport entre la partie hydrophile et la partie lipophile dans la molécule. Le terme HLB est bien connu de l'homme du métier et est décrit par exemple dans "The HLB system. A time-saving guide to Emulsifier Selection" (published by ICI Americas Inc ; 1984). Pour les émulsionnants, le HLB va généralement de 3 à 8 pour la préparation des émulsions E/H et de 8 à 18 pour la préparation des émulsions H/E, alors que les tensioactifs moussants ont généralement un HLB supérieur à 20.

Les tensioactifs moussants non ioniques de la composition de l'invention peuvent être choisis notamment parmi les alkyl polyglucosides (APG), les esters de glycérol oxyalkylénés, les esters de sucre oxyalkylénés, et leurs mélanges. Ce sont de manière préférée des APG.

Comme alkylpolyglucosides, on utilise de préférence ceux contenant un groupe alkyle comportant de 6 à 30 atomes de carbone et de préférence de 8 à 16 atomes de carbone, et contenant un groupe glucoside comprenant de préférence 1,2 à 3 unités de glucoside. Les alkylpolyglucosides peuvent être choisis par exemple parmi le decylglucoside (Alkyl-C9/C11-polyglucoside (1.4)) comme le produit commercialisé sous la dénomination Mydol 10 ® par la société Kao Chemicals ou le produit commercialisé sous la dénomination Plantacare 2000 UP® par la société Cognis ; le caprylyl/capryl glucoside comme le produit commercialisé sous la dénomination Plantacare KE 3711® par la société Cognis ; le laurylglucoside comme le produit commercialisé sous la dénomination Plantacare 1200 UP® par la société Cognis ; le cocoglucoside comme le produit commercialisé sous la dénomination Plantacare 818 UP® par la société Cognis ; le caprylylglucoside comme le produit commercialisé sous la dénomination Plantacare 810 UP® par la société Cognis ; et leurs mélanges.

Les esters de glycérol oxyalkylénés sont notamment les dérivés polyoxyéthylénés des esters de glycéryle et d'acide gras et de leurs dérivés hydrogénés. Ces esters de glycérol oxyalkylénés peuvent être choisis par exemple parmi les esters de glycéryle et d'acides gras hydrogénés et oxyéthylénés tel que le PEG-200 hydrogenated glyceryl palmate commercialisé sous la dénomination Rewoderm LIS 80 par la société Goldschmidt ; les cocoates de glycéryle oxyéthylénés comme le PEG-7 glyceryl cocoate commercialisé sous la dénomination Tegosoft GC par la société Goldschmidt, et le PEG-30 glyceryl cocoate commercialisé sous la dénomination Rewoderm LI-63 par la société Goldschmidt ; et leurs mélanges.

Les esters de sucres oxyalkylénés sont notamment les éthers de polyéthylène glycol des esters d'acide gras et de sucre. Ces esters de sucre oxyalkylénés peuvent être choisis par exemple parmi les esters de glucose oxyéthylénés tels que le PEG-120 methyl glucose dioleate commercialisé sous la dénomination Glucamate DOE 120 par la société Amerchol.

Selon un mode préféré de réalisation de l'invention, le tensioactif non ionique est un alkylpolyglucoside qui peut être choisi notamment parmi le decylglucoside, le caprylyl/capryl glucoside, le laurylglucoside, le cocoglucoside, le caprylylglucoside, et leurs mélanges.

La composition selon l'invention contient une quantité de tensioactif(s) moussant(s) non ionique(s) (en matière active) pouvant aller par exemple de 1 à 50 % en poids, de préférence de 1 à 40 % en poids et mieux de 2 à 30 % en poids par rapport au poids total de la composition.

La composition selon l'invention peut contenir, outre les tensioactifs moussants non ioniques, un ou plusieurs tensioactifs moussants choisis parmi les tensioactifs anioniques, amphotères et zwitterioniques.

### Tensioactifs anioniques

Les tensioactifs moussants anioniques pouvant être ajoutés dans la composition selon l'invention peuvent être choisis notamment parmi les dérivés anioniques de protéines d'origine végétale ou de protéines de soie, les phosphates et alkylphosphates, les carboxylates, les sulfosuccinates, les dérivés des aminoacides, les alkyl sulfates, les alkyl éther sulfates, les sulfonates, les iséthionates, les taurates, les alkyl sulfoacétates, les polypeptides, les dérivés anioniques d'alkyl polyglucoside, et leurs mélanges.

Les dérivés anioniques de protéines d'origine végétale sont des hydrolysats de protéine à groupement hydrophobe, ledit groupement hydrophobe pouvant être naturellement présent dans la protéine ou être ajouté par réaction de la protéine et/ou de l'hydrolysat de protéine avec un composé hydrophobe. Les protéines sont d'origine végétale ou dérivées de soie, et le groupement hydrophobe peut être notamment une chaîne grasse, par exemple une chaîne alkyle comportant de 10 à 22 atomes de carbone. Comme dérivés anioniques de protéines d'origine végétale, on peut plus particulièrement citer les hydrolysats de protéines de pomme, de blé, de soja, d'avoine, comportant une chaîne alkyle ayant de 10 à 22 atomes de carbone et leurs sels. La chaîne alkyle peut être notamment une chaîne lauryle et le sel peut être un sel de sodium, de potassium et/ou d'ammonium.

Ainsi, comme hydrolysats de protéine à groupement hydrophobe, on peut citer par exemple les sels des hydrolysats de protéine de soie modifiée par l'acide laurique, tels que le produit commercialisé sous la dénomination KAWA SILK par la société Kawaken ; les sels des hydrolysats de protéine de blé modifiée par l'acide laurique, tels que le sel de potassium commercialisé sous la dénomination Aminofoam W OR par la société Croda (nom CTFA : Potassium lauroyl wheat aminoacids) et le sel de sodium commercialisé sous la dénomination PROTEOL LW 30 par la société Seppic (nom CTFA : sodium lauroyl wheat aminoacids) ; les sels des hydrolysats de protéine d'avoine comportant une chaîne alkyle ayant de 10 à 22 atomes de carbone, et plus spécialement les sels des hydrolysats de protéine d'avoine modifiée par l'acide laurique, tels que le sel de sodium commercialisé sous la dénomination PROTEOL OAT (soltion aqueuse à 30%) par la société Seppic (nom CTFA : Sodium lauroyl oat aminoacids) ; les sels des hydrolysats de protéine de pomme, comportant une chaîne alkyle ayant de 10 à 22 atomes de carbone, tels que le sel de sodium commercialisé sous la dénomination PROTEOL APL (solution hydroglycolique à 30%) par la société Seppic (nom CTFA : Sodium Cocoyl Apple amino acids). On peut citer aussi le mélange de lauroyl-aminoacides (aspartique, glutamique, glycine, alanine) neutralisé au N-methylglycinate de sodium, commercialisé sous la dénomination PROTEOL SAV 50 S par la société Seppic (nom CTFA : Sodium Cocoyl amino acids).

Comme phosphates et alkylphosphates, on peut citer par exemple les monoalkylphosphates et les dialkyl phosphates, tels que le mono-phosphate de lauryle commercialisé sous la dénomination MAP 20® par la société Kao Chemicals, le sel de potassium de l'acide dodécyl-phosphorique, mélange de mono- et di-ester (diester majoritaire) commercialisé sous la dénomination CRAFOL AP-31® par la société Cognis, le mélange de monoester et de di-ester d'acide octylphosphorique, commercialisé sous la dénomination CRAFOL AP-20® par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER® par la société Condea, le sel de potassium ou de triéthanolamine de mono-alkyl (C12-C13) phosphate commercialisé sous les références ARLATONE MAP 230K-40® et ARLATONE MAP 230T-60® par la société Uniqema, le lauryl phosphate de potassium commercialisé sous la dénomination DERMALCARE MAP XC-99/09® par la société Rhodia Chimie, et le cétylphosphate de potassium commercialisé sous la dénomination ARLATONE MAP 160K par la société Uniqema.

Comme carboxylates, on peut citer :
- Les amido éthercarboxylates (AEC), comme le Lauryl amido ether carboxylate de sodium (3 OE), commercialisé sous la dénomination AKYPO FOAM 30® par la société Kao Chemicals.
- les sels d'acides carboxyliques polyoxyéthylénés, comme le Lauryl ether carboxylate de sodium (C₁₂₋₁₄₋₁₆ 65/25/10) oxyéthyléné (6 OE) commercialisé sous la dénomination AKYPO SOFT 45 NV® par la société Kao Chemicals, les acides gras d'origine huile d'olive polyoxyéthylénés et carboxyméthylés commercialisés sous la dénomination OLIVEM 400® par la société BIOLOGIA E TECNOLOGIA, le tri-decyl ether carboxylate de sodium oxyéthyléné (6 OE) commercialisé sous la dénomination NIKKOL ECTD-6NEX ® par la société Nikkol.
- les sels d'acides gras (savons) ayant une chaîne alkyl en C6 à C22, neutralisés par une base organique ou minérale telle que la potasse, la soude, la triéthanolamine,la N-methyl glucamine, la lysine et l'arginine.

Comme dérivés des aminoacides, on peut citer notamment les sels alcalins d'aminoacides, tels que :
- les sarcosinates, comme le Lauroyl sarcosinate de sodium commercialisé sous la dénomination SARKOSYL NL 97® par la société Ciba ou commercialisé sous la dénomination ORAMIX L 30® par la société Seppic, le myristoyl sarcosinate de sodium commercialisé sous la dénomination NIKKOL SARCOSINATE MN® par la société Nikkol, le palmitoyl sarcosinate de sodium commercialisé sous la dénomination NIKKOL SARCOSINATE PN ® par la société Nikkol.
- les alaninates, comme le N-lauroyl-N methyl amidopropionate de sodium commercialisé sous la dénomination SODIUM NIKKOL ALANINATE LN 30® par la société Nikkol, ou commercialisé sous la dénomination ALANONE ALE® par la société Kawaken, le N-lauroyl N-methyl alanine triéthanolamine commercialisé sous la dénomination ALANONE ALTA ® par la société Kawaken.
- Les glutamates, comme le mono-cocoyl glutamate de triéthanolamine commercialisé sous la dénomination ACYLGLUTAMATE CT-12® par la société Ajinomoto, le lauroylglutamate de triéthanolamine commercialisé sous la dénomination ACYLGLUTAMATE LT-12® par la société Ajinomoto.
- les aspartates, comme le mélange de N-lauroyl aspartate de triethanolamine /N-myristoyl aspartate de triethanolamine commercialisé sous la dénomination ASPARACK ® par la société Mitsubishi.
- les dérivés de glycine (glycinates), comme le N-cocoyl glycinate de sodium commercialisé sous les dénominations AMILITE GCS-12® et AMILITE GCK 12 par la société Ajinomoto.
- Les citrates tels que le mono-ester citrique d'alcools de coco oxyéthylénés (9 moles), commercialisé sous la dénomination WITCONOL EC 1129 par la société Goldschmidt.
- les galacturonates tels que le dodeécyl d-galactoside uronate de sodium commercialisé par la société Soliance.

Comme sulfosuccinates, on peut citer par exemple le mono-sulfosuccinate d'alcool laurylique (C12/C14 70/30) oxyéthyléné (3 OE) commercialisé sous les dénominations SETACIN 103 SPECIAL®, REWOPOL SB-FA 30 K 4® par la société Witco, le sel di-sodique d'un hemi-sulfosuccinate des alcools C12-C14, commercialisé sous la dénomination SETACIN F SPECIAL PASTE® par la société Zschimmer Schwarz, l'oléamidosulfosuccinate di-sodique oxyéthyléné (2 OE) commercialisé sous la dénomination STANDAPOL SH 135® par la société Cognis, le mono-sulfosuccinate d'amide laurique oxyéthyléné (5 OE) commercialisé sous la dénomination LEBON A-5000® par la société Sanyo, le sel di-sodique de mono-sulfosuccinate de lauryl citrate oxyéthyléné (10 OE) commercialisé sous la dénomination REWOPOL SB CS 50® par la société Witco, le mono-sulfosuccinate de mono-éthanolamide ricinoléique commercialisé sous la dénomination REWODERM S 1333® par la société Witco. On peut utiliser aussi les sulfosuccinates de polydimethylsiloxane tels que le disodium PEG-12 dimethicone sulfosuccinate commercialisé sous la dénomination MACKANATE-DC30 par la société Mac Intyre.

Comme alkyl sulfates, on peut citer par exemple le lauryl sulfate de triéthanolamine (nom CTFA : TEA-lauryl sulfate) tel que le produit commercialisé par la société Huntsman sous la dénomination EMPICOL TL40 FL ou celui commercialisé par la société Cognis sur la dénomination TEXAPON T42, produits qui sont à 40% en solution aqueuse. On peut aussi citer le lauryl sulfate d'ammonium (nom CFTA : Ammonium lauryl sulfate) tel que le produit commercialisé par la société Huntsman sous la dénomination EMPICOL AL 30FL qui est à 30% en solution aqueuse.

Comme alkyl éther sulfates, on peut citer par exemple le lauryl éther sulfate de sodium (nom CTFA : sodium laureth sulfate) comme celui commercialisé sous les dénominations TEXAPON N40 et TEXAPON AOS 225 UP par la société Cognis, le lauryl éther sulfate d'ammonium (nom CTFA : ammonium laureth sulfate) comme celui commercialisé sous la dénomination STANDAPOL EA-2 par la société Cognis.

Comme sulfonates, on peut citer par exemple les alpha-oléfines sulfonates comme l'alpha-oléfine sulfonate de sodium (C14-16) commercialisé sous la dénomination BIO-TERGE AS-40® par la société Stepan, commercialisé sous les dénominations WITCONATE AOS PROTEGE® et SULFRAMINE AOS PH 12® par la société Witco ou commercialisé sous la dénomination BIO-TERGE AS-40 CG® par la société Stepan, l'oléfine sulfonate de sodium secondaire commercialisé sous la dénomination HOSTAPUR SAS 30® par la société Clariant ; les alkyl aryl sulfonates linéaires comme le xylène sulfonate de sodium commercialisé sous les dénominations MANROSOL SXS30®, MANROSOL SXS40®, MANROSOL SXS93® par la société Manro.

Comme iséthionates, on peut citer les acyliséthionates comme le cocoyl-iséthionate de sodium, tel que le produit commercialisé sous la dénomination JORDAPON CI P® par la société Jordan.

Comme taurates, on peut citer le sel de sodium de méthyltaurate d'huile de palmiste commercialisé sous la dénomination HOSTAPON CT PATE® par la société Clariant ; les N-acyl N-méthyltaurates comme le N-cocoyl N-methyltaurate de sodium commercialisé sous la dénomination HOSTAPON LT-SF® par la société Clariant ou commercialisé sous la dénomination NIKKOL CMT-30-T® par la société Nikkol, le palmitoyl methyltaurate de sodium commercialisé sous la dénomination NIKKOL PMT® par la société Nikkol.

Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET® par la société Cesalpinia, le sel di-sodique d'ester sulfosuccinique de cocoylpolyglucoside (1,4), commercialisé sous la dénomination ESSAI 512 MP® par la société Seppic, le sel de sodium d'ester citrique de cocoyl polyglucoside (1,4) commercialisé sous la dénomination EUCAROL AGE-EC® par la société Cesalpinia.

### Tensioactifs moussants amphotères et zwitterioniques

Les tensioactifs amphotères et zwitterioniques peuvent être choisis par exemple parmi les bétaïnes, les N-alkylamidobétaïnes et leurs dérivés, les sultaïnes, les alkyl polyaminocarboxylates, les alkylamphoacétates et leurs mélanges.

Comme bétaïnes, on peut citer notamment les alkylbétaïnes comme par exemple la cocobétaïne comme le produit commercialisé sous la dénomination DEHYTON AB-30® par la société Cognis, la laurylbétaïne comme le produit commercialisé sous la dénomination GENAGEN KB® par la société Clariant, la laurylbétaïne oxyethylénée (10 OE), comme le produit commercialisé sous la dénomination LAURYLETHER (10 OE) BETAINE® par la société Shin Nihon Rica, la stéarylbétaïne oxyéthylénée (10 OE) comme le produit commercialisé sous la dénomination STEARYLETHER (10 OE) BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou commercialisée sous la dénomination EMPIGEN BB® par la société Albright & Wilson, la lauramidopropyl bétaïne commercialisée sous la dénomination REWOTERIC AMB12P® par la société Witco.

Comme sultaines, on peut citer les hydroxylsultaïnes, telles que la Cocamidopropyl hydroxysultaïne comme le produit commercialisé sous la dénomination REWOTERIC AM CAS par la société Golschmidt-Degussa, ou le produit commercialisé sous la dénomination CROSULTAINE C-50® par la société Croda.

Come alkyl polyaminocarboxylates (APAC), on peut citer le cocoylpolyamino-carboxylate de sodium, commercialisé sous la dénomination AMPHOLAK 7 CX/C®,et AMPHOLAK 7 CX® par la société Akzo Nobel, le stéarylpolyamidocarboxylate de sodium commercialisé sous la dénomination AMPHOLAK 7 TX/C par la société Akzo Nobel, la carboxyméthyloléylpolypropylamine de sodium, commercialisé sous la dénomination AMPHOLAK XO7/C® par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocoamphodiacetate) comme le produit commercialisé sous la dénomination MIRANOL C2M CONCENTRE NP® par la société Rhodia, le N-cocoyl-N-hydroxyéthyl-N-carboxyméthyl-éthylènediamine N-sodique (nom CTFA : sodium cocamphoacetate), le cocoamphohydroxypropyl sulfonate de sodium commercialisé sous la dénomination MIRANOL CSE par la société Rhodia.

Les tensioactifs moussants anioniques, amphotères et zwitterioniques, quand ils sont présents dans la composition de l'invention, peuvent être en une quantité (en matière active) allant par exemple de 0,5 à 15 % en poids, et de préférence de 1 à 10 % en poids par rapport au poids total de la composition.

Selon un mode préféré de réalisation de l'invention, la composition contient comme tensioactif moussant autre que le tensioactif non ionique, au moins un dérivé anionique de protéines d'origine végétale ou de protéines de soie.

Selon un autre mode préféré de réalisation de l'invention, la composition de l'invention contient au moins un alkylpolyglucoside et au moins un dérivé anionique de protéines d'origine végétale ou de protéines de soie. La composition selon l'invention contenant le dérivé d'urée hydroxylé, le polymère revendiqué et cette association de tensioactifs a l'avantage d'être très douce et très bien tolérée, tout en étant bien hydratante et en moussant bien.

### Sel d'ammonium quaternaire polymérique

Les sels d'ammonium quaternaire polymériques sont des polymères cationiques ou amphotères contenant au moins un atome d'azote quaternisé. Comme sels d'ammonium quaternaire polymériques, on peut citer notamment les Polyquaternium (nom CTFA), qui apportent douceur et onctuosité à la crème moussante. Ces polymères peuvent être choisis de préférence parmi les polymères suivants :
- Polyquaternium 5 tel que le produit MERQUAT 5 commercialisé par la société CALGON ;
- Polyquaternium 6 tel que le produit SALCARE SC 30 commercialisé par la société CIBA, et le produit MERQUAT 100 commercialisé par la société CALGON ;
- Polyquaternium 7 tel que les produits MERQUAT S, MERQUAT 2200 et MERQUAT 550 commercialisés par la société CALGON, et le produit SALCARE SC 10 commercialisé par la société CIBA ;
- Polyquaternium 10 tel que le produit Polymer JR400 commercialisé par la société AMERCHOL ;
- Polyquaternium 11 tel que les produits GAFQUAT 755, GAFQUAT 755N et GAFQUAT 734 commercialisés par la société ISP ;
- Polyquaternium 15 tel que le produit ROHAGIT KF 720 F commercialisé par la société ROHM ;
- Polyquaternium 16 tel que les produits LUVIQUAT FC905, LUVIQUAT FC370, LUVIQUAT HM552 et LUVIQUAT FC550 commercialisés par la société BASF ;
- Polyquaternium 22 tel que le produit MERQUAT 280 commercialisé par la société CALGON ;
- Polyquaternium 28 tel que le produit STYLEZE CC10 commercialisé par la société ISP ;
- Polyquaternium 39 tel que le produit MERQUAT PLUS 3330 commercialisé par la société CALGON ;
- Polyquaternium 44 tel que le produit LUVIQUAT CARE commercialisé par la société BASF ;
- Polyquaternium 46 tel que le produit LUVIQUAT HOLD commercialisé par la société BASF ;
- Polyquaternium 47 tel que le produit MERQUAT 2001 commercialisé par la société CALGON.

De manière préférée, les sels d'ammonium quaternaire sont choisis parmi le Polyquaternium-7, le Polyquaternium-10, le Polyquaternium-39, le Polyquaternium-47, et leurs mélanges.

Les sels d'ammonium quaternaire polymériques peuvent être en une quantité (en matière active) allant par exemple de 0,05 à 10 % en poids et mieux de 0,1 à 5 % en poids par rapport au poids total de la composition.

### Adjuvants

Comme indiqué ci-dessus, le milieu aqueux de la composition selon l'invention peut contenir, outre l'eau, un ou plusieurs solvants hydrosolubles choisis parmi les alcools inférieurs (mono-alcools) comportant de 1 à 6 atomes de carbone, tels que l'éthanol ; et les polyols tels que la glycérine, les glycols comme le butylène glycol, l'isoprène glycol, le propylène glycol, les polyéthylène glycols tels que le PEG-8 ; le sorbitol ;les sucres tels que le glucose, le fructose, le maltose, le lactose, le sucrose ; et leurs mélanges, dans la mesure où ces composés n'altèrent pas les propriété recherchées de la composition selon l'invention. La quantité de solvant(s) dans la composition de l'invention peut aller par exemple de 0,1 à 10 % en poids, de préférence de 0,5 à 5 % en poids, mieux de 0,5 à 5 % en poids et encore mieux de 0,5 à 3 % en poids par rapport au poids total de la composition.

Les compositions de l'invention peuvent contenir aussi des adjuvants habituellement utilisés dans le domaine cosmétique. Comme adjuvants, on peut citer par exemple les parfums, les conservateurs, les séquestrants (EDTA), les pigments, les charges notamment exfoliantes, les colorants solubles, les filtres solaires, les actifs cosmétiques ou dermatologiques tels que les hydratants comme l'acide hyaluronique ; les céramides ; les vitamines hydrosolubles ou liposolubles comme la vitamine C et ses dérivés tels que la vitamine CG ; les antiseptiques ; les antiséborrhéïques ; les antimicrobiens tels que le peroxyde de benzoyle, l'acide salicylique, le triclosan, l'acide azélaïque ; les azurants optiques. Les quantités de ces différents adjuvants sont celles classiquement utilisées dans le domaine considéré, et par exemple de 0,01 à 5 % du poids total de la composition. Ces adjuvants ainsi que leurs concentrations doivent être tels qu'ils ne modifient pas la propriété recherchée pour la composition de l'invention.

Comme exfoliants, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. Ces particules peuvent être présentes en une quantité allant par exemple de 0,5 à 40 % en poids, de préférence de 1 à 20 % en poids et mieux de 1 à 10 % en poids par rapport au poids total de la composition. Quand la composition contient des particules exfoliantes, elle peut constituer notamment une composition de gommage de la peau du visage ou du corps.

Les compositions de l'invention peuvent contenir aussi des polymères, notamment anioniques et non ioniques.

Comme polymères anioniques, on peut citer notamment ceux comportant au moins une chaîne hydrophobe, et en particulier ceux dérivés d'acide acrylique ou méthacrylique, comme le copolymère acrylates/steareth-20 methacrylate commercialisé sous la dénomination Aculyn 22 par la société Rohm & Haas (nom CTFA : Acrylates/Steareth-30 Methacrylate copolymer) ; le terpolymère acide (méth)acrylique / acrylate d'éthyle / méthacrylate de béhényle oxyéthyléné (25 OE), en émulsion aqueuse commercialisé sous la dénomination Aculyn 28 par la société Rohm & Haas; le copolymère acide acrylique/itaconate de mono-cétyle oxyéthyléné (20 OE), en dispersion aqueuse à 30 % commercialisé sous la dénomination Structure 3001 par la société National Starch ; le copolymère acide acrylique/itaconate de mono-stéaryle oxyéthyléné (20 OE) en dispersion aqueuse à 30 % commercialisé sous la dénomination Structure 2001 par la société National Starch ; le copolymère acrylates/acrylate modifié par des alcools en C12-C24 polyoxyéthylénés (25 OE), sous forme de latex à 30-32 % de copolymère, commercialisé sous la dénomination Synthalen W2000 par la société 3V SA, ainsi que les copolymères vendus sous les noms PEMULEN ou CARBOPOL par la Société Noveon, comme le copolymère acrylate/C₁₀-C₃₀-alkylacrylate tel que les produits PEMULEN TR1, PEMULEN TR2 ou CARBOPOL 1382 (nom CTFA : Acrylates/C10-30 Alkyl acrylate Crosspolymer).

La composition peut contenir aussi des polymères comportant au moins un monomère à groupement sulfonique, et notamment les polymères et copolymères d'acide 2-acrylamido-2-méthylpropane sulfonique (AMPS) tels que :
- l'homopolymère réticulé et neutralisé d'acide 2-acrylamido 2-méthylpropane sulfonique, commercialisé par la société Clariant sous la dénomination commerciale « Hostacerin AMPS » (nom CTFA : ammonium polyacryldimethyltauramide)
- les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom C.T.F.A. : Polyacrylamide / C13-14 Isoparaffin / Laureth-7), sous le nom de SIMULGEL 600 par la société Seppic (nom C.T.F.A. : Acrylamide / Sodium acryloyldimethyltaurate copolymer /Isohexadecane / Polysorbate 80),
- les copolymères d'acide(méth)acrylique ou de (méth)acrylate et d'acide 2-acrylamido- 2-méthylpropane sulfonique, notamment ceux se présentant sous la forme d'une émulsion E/H, tels que ceux commercialisés sous le nom de SIMULGEL NS par la société Seppic (copolymère d'acrylamido-2-methyl propane sulfonate de sodium / hydroxyethyl acrylate en émulsion inverse à 40% dans Polysorbate 60 et squalane) (nom CTFA : hydroxyethyl acrylate/sodium acryloyldimethyltaurate copolymer / squalane / polysorbate 60) ou ceux commercialisés sous le nom de SIMULGEL EG par la société Seppic (copolymère d'acide acrylique / acrylamido-2-methylpropane-sulfonique sous forme de sel de sodium en émulsion inverse à 45% dans isohexadecane / eau) (nom C.T.F.A. : Sodium Acrylate / Sodium acryloydimethyl-taurate copolymer / Isohexadecane /Polysorbate 80),
- les copolymères d'acide 2-acrylamido-2-méthylpropane sulfonique et de vinylpyrrolidone ou de vinylformamide, tels que les produits commercialisés sous les dénominations ARISTOFLEX AVC par la société Clariant,
- les polymères d'AMPS modifiés hydrophobes comme notamment le copolymère d'AMPS et de methacrylate d'alcool en C12-C14 éthoxylé (copolymère non réticulé obtenu à partir de Genapol LA-070 et d'AMPS) (nom CTFA : Ammonium Acryloyldimethyltaurate / Laureth-7 Methacrylate Copolymer) commercialisé sous la dénomination ARISTOFLEX LNC par la société Clariant, et le copolymère d'AMPS et de méthacrylate de stéaryle éthoxylé (25 EO) (copolymère réticulé par le trimethylolpropane triacrylate, obtenu à partir de Genapol T-250 et d'AMPS) (nom CTFA : Ammonium Acryloyldimethyltaurate / Steareth-25 Methacrylate Crosspolymer) commercialisé sous la dénomination ARISTOFLEX HMS par la société Clariant.

Ces polymères peuvent être en une quantité (en matière active) allant par exemple de 0,05 à 10 % en poids et mieux de 0,1 à 5 % en poids par rapport au poids total de la composition.

Les compositions selon l'invention peuvent être utilisées sur toutes les matières kératiniques telles que la peau, le cuir chevelu, les cheveux, les cils, les sourcils, les ongles ou les muqueuses, notamment comme produit d'hygiène, par exemple comme produit de nettoyage de la peau, des muqueuses et/ou des cheveux, en particulier comme produit de nettoyage et/ou de démaquillage de la peau (du visage et/ou du corps), comme produit de douche (produit deux-en-un), comme shampoing et après-shampoing, comme produit de rasage, comme masque à rincer, et comme produit exfoliant (appelé aussi desquamant ou désincrustant) aussi bien pour le visage que pour le corps ou pour les mains, après addition de particules exfoliantes.

L'invention a encore pour objet l'utilisation cosmétique de la composition telle que définie ci-dessus, comme produit de nettoyage et/ou de démaquillage de la peau, comme produit de douche, comme shampoing, comme produit après-shampoing, comme produit de rasage, comme masque à rincer, comme produit exfoliant.

Un autre objet de l'invention est un procédé de nettoyage d'une matière kératinique, telle que la peau, du cuir chevelu, des cheveux, des cils, des sourcils, des ongles ou des muqueuses, caractérisé par le fait qu'on applique sur la matière kératinique, une composition telle que définie ci-dessus, et qu'on la rince.

La matière kératinique est de préférence la peau.

Les compositions selon l'invention peuvent être par exemple utilisées de la manière suivante :
1) quand les compositions selon l'invention constituent des produits de nettoyage doux pour le visage, elles peuvent être utilisées de la manière suivante :
   - on fait mousser le produit dans les mains avec de l'eau
   - on applique la mousse sur le visage
   - on nettoie le visage
   - on rince à l'eau
2) quand les compositions selon l'invention constituent des produits démaquillants, elles peuvent être utilisées comme indiqué ci-dessus mais elles peuvent aussi être appliquées à sec sur le visage, puis massées jusqu'à obtention d'un démaquillage satisfaisant, et rincées à l'eau, ou bien elles peuvent être appliquées au moyen d'un coton.
3) on peut les utiliser comme produit de douche deux-en-un, comme shampooing et/ou après shampooing, comme masque à rincer ou comme produit de rasage, de la manière habituelle d'utilisation de ces produits.

Les exemples qui suivent servent à illustrer l'invention sans toutefois présenter un caractère limitatif. Les quantités indiquées sont en % en poids sauf mention contraire, et elles correspondent sauf mention contraire à la quantité de matière première et non à la quantité de matière active. Les noms des composés utilisés sont indiqués en nom CTFA, en nom chimique ou en nom commercial.

### Exemple 1 selon l'invention

| Composition moussante | Exemple 1 |
|---|---|
| | |

| *PHASE I* | |
|---|---|
| Eau | Qsp 100 % |
| Hydrovance (1) (soit 7 % de matière active) | 14 % |
| Conservateur | 0,3 % |
| Disodium EDTA | 0,2 % |
| Aculyn 22 (2) | 2,2 % |
| Carbopol 1382 | 0,4% |
| Hostacerin AMPS | 0,6 % |
| Triéthanolamine | 0,7 % |
| | |

| *PHASE II* | |
|---|---|
| Decyl glucoside (3) | 5 % |
| Coco-glucoside (4) | 3 % |
| Ammonium lauryl Sulfate (5) | 2 % |
| TEA-lauryl sulfate (6) | 0,5 % |
| Sodium Lauroyl OAT aminoacids (7) | 2 % |
| Triéthanolamine | 0,01 % |
| | |

| *PHASE lii* | |
|---|---|
| Poylquaternium-47 | 0.3 % |
| parfum | 0.1 % |

| | |
|---|---|
| (1) Hydrovance = N-(2-hydroxyéthyl)-urée à 50% en poids en solution aqueuse | |
| (2) Copolymère acrylique (en dispersion aqueuse à 30 % de matière active) | |
| (3) ORAMIX NS 10 (solution aqueuse à 55% de matière active) | |
| (4) PLANTACARE 1200 UP (solution aqueuse à 50% de matière active) | |
| (5) EMPICOL AL 30/FL (solution aqueuse à 30%) | |
| (6) EMPICOL TL 40/FL (solution aqueuse à 40%) | |
| (7) PROTEOL OAT (solution à 30%) | |

Mode opératoire : on prépare la phase 1, on y ajoute peu à peu les composés de la phase II, puis ensuite la phase III.

On obtient une composition qui mousse bien, qui donne une mousse qui démarre bien et est abondante. En outre, la composition se rince bien et elle est bien tolérée par la peau. Elle peut être avantageusement utilisée pour le nettoyage de la peau du visage.

Exemple 1 comparatif : on remplace dans l'exemple 1, l'hydroxyéthylurée par la glycérine. On obtient une composition qui donne une mousse qui démarre plus lentement, qui est beaucoup moins abondante et moins dense que celle de l'exemple 1 avec l'hydroxyethyl urée. En outre, le rinçage est alors moins rapide.

### Exemple 2 selon l'invention

| Composition/ moussante exfoliante | Exemple 2 |
|---|---|
| *PHASE I* | |
| Eau | Qsp 100 % |
| Hydrovance (1) (soit 10% de matière active) | 20 % |
| Conservateur | 0.3 % |
| Disodium EDTA | 0.25 % |
| Aculyn 22 (2) | 3 % |
| Laponite | 1 % |
| Triéthanolamine | 0.7 % |
| | |

| *PHASE II* | |
|---|---|
| Decylglucoside (3) | 7 % |
| Lauroyl-glucoside (4) | 1 % |
| Sodium cocoyl apple aminoacids (5) | 6 % |
| PEG-60 Hydrogenated castor oil | 0.8 % |
| Sodium laureth sulfate (6) | 3 % |
| | |

| *PHASE III* | |
|---|---|
| Alumine | 4 % |
| Billes de polyéthylène | 3 % |
| Pierre ponce | 0.5 % |
| | |

| *PHASE IV* | |
|---|---|
| Benzophenone-4 (filtre) | 0.15 % |
| Parfum | 0.1 % |

| | |
|---|---|
| (1) Hydrovance = N-(2-hydroxyéthyl)-urée à 50% en poids en solution aqueuse | |
| (2) Copolymère acrylique (en dispersion aqueuse à 30 % de matière active) | |
| (3) ORAMIX NS 10 (solution aqueuse à 55% de matière active) | |
| (4) PLANTACARE 1200 UP (solution aqueuse à 50% de matière active) | |
| (5) PROTEOL APL (solution aqueuse à 30% de matière active) | |
| (6) TEXAPON N40 (solution aqueuse à 28 % de matière active) | |

Le mode opératoire est analogue à celui de l'exemple 1.

On obtient une composition qui donne une mousse qui démarre bien et qui est abondante. En outre, la composition se rince bien et laisse la peau douce. Elle constitue une composition de gommage très bien tolérée.

## Revendications

1. Composition de nettoyage contenant dans un milieu aqueux, (1) au moins un tensioactif moussant non ionique et (2) au moins un composé de formule (I) suivante : 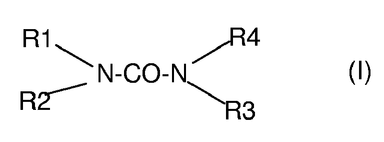 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle, ainsi que leurs sels, leurs solvats, et leurs isomères, et (3) au moins un sel d'ammonium quaternaire polymérique.

2. Composition selon la revendication 1, **caractérisée en ce qu'**elle comprend au moins 20 % en poids d'eau par rapport au poids total de la composition.

3. Composition selon la revendication 1 ou 2, **caractérisée en ce que**, dans les composés de formule (I), R1 désigne un groupe hydroxyalkyle en C2-C6 et R2, R3, R4 désignent, indépendamment l'un de l'autre, un atome d'hydrogène ou un groupe alkyle en C1-C4.

4. Composition selon l'une quelconque des revendications précédentes, **caractérisée en ce que**, dans les composés de formule (I), R1 désigne un groupe hydroxyalkyle en C2-C6 comprenant de 1 à 5 groupes hydroxyles, et R2, R3, R4 désignent un atome d'hydrogène.

5. Composition selon la revendication précédente, **caractérisée en ce que** R1 désigne un groupe hydroxyalkyle en C2-C6 comprenant 1 groupe hydroxyle.

6. Composition selon l'une quelconque des revendications précédentes, **caractérisée en ce que**, dans les composés de formule (I), R1 désigne un groupe hydroxyalkyle en C2-C4 comprenant 1 groupe hydroxyle et R2, R3, R4 désignent un atome d'hydrogène.

7. Composition selon l'une quelconque des revendications précédentes, **caractérisée en ce que** le composé de formule (I) est choisi parmi le N-(2-hydroxyéthyl)-urée ; le N-(2-hydroxypropyl)-urée ; le N-(3-hydroxypropyl)-urée ; le N-(2,3-dihydroxypropyl)-urée ; le N-(2,3,4,5,6-pentahydroxyhexyl)-urée ; le N-méthyl-N-(1,3,4,5,6-pentahydroxy-2-hexyl)-urée ; le N-méthyl-N'-(1-hydroxy-2-méthyl-2-propyl)-urée ; le N-(1-hydroxy-2-méthyl-2-propyl)-urée ; le N-(1,3-dihydroxy-2-propyl)-urée ; le N-(tris-hydroxyméthyl-méthyl)-urée ; le N-éthyl-N'-(2-hydroxyéthyl)-urée ; le N,N-bis-(2-hydroxyéthyl)-urée ; le N,N'-bis-(2-hydroxyéthyl)-urée ; le N,N-bis-(2-hydroxypropyl)-urée ; le N,N'-Bis-(2-hydroxypropyl)-urée ; le N,N-Bis-(2-hydroxyéthyl)-N'-propyl-urée ; le N,N-Bis-(2-hydroxypropyl)-N'-(2-hydroxyéthyl)-urée ; le N-tert.Butyl-N'-(2-(hydroxyéthyl)-N'-(2-(hydroxypropyl)-urée; le N-(1,3-dihydroxy-2-propyl)-N'-(2-hydroxyéthyl)-urée ; le N,N-Bis-(2-hydroxyéthyl)-N',N'-dimethyl-urée ; le N,N,N',N'-tetrakis-(2-hydroxy-éthyl)-urée ; le N',N'-Bis-(2-hydroxyéthyl)-N',N'-bis-(2-hydroxypropyl)-urée ; et leurs mélanges.

8. Composition selon l'une quelconque des revendications précédentes, **caractérisée en ce que** le composé de formule (I) est le N-(2-hydroxyéthyl)-urée.

9. Composition selon l'une quelconque des revendications précédentes, **caractérisée en ce que** les sels des composés de formule (1) sont choisis parmi les sels de l'acide sulfurique, l'acide chlorhydrique, l'acide bromhydrique, l'acide iodhydrique, l'acide phosphorique, l'acide borique, l'acide propionique, l'acide acétique, l'acide téréphtalique, l'acide citrique, l'acide tartrique.

10. Composition selon l'une quelconque des revendications précédentes, **caractérisée en ce que** le composé de formule (1) est présent en une quantité allant de 0,5 à 30 % en poids par rapport au poids total de la composition.

11. Composition selon l'une quelconque des revendications précédentes, **caractérisée en ce que** le tensioactif moussant non ionique est choisi parmi les alkylpolyglucosides, les esters de glycérol oxyalkylénés, les esters de sucre oxyalkylénés, et leurs mélanges.

12. Composition selon la revendication précédente, **caractérisée en ce que** le tensioactif moussant non ionique est choisi parmi le decylglucoside, le caprylyl/capryl glucoside, le laurylglucoside, le cocoglucoside, le caprylylglucoside, et leurs mélanges.

13. Composition selon l'une quelconque des revendications précédentes, **caractérisée en ce que** la quantité de tensioactif(s) moussant(s) non ionique(s) va de 1 à 50 % en poids par rapport au poids total de la composition.

14. Composition selon l'une quelconque des revendications précédentes, **caractérisée en ce qu'**elle contient en outre un ou plusieurs tensioactifs moussants choisis parmi les tensioactifs anioniques, amphotères et zwitterioniques.

15. Composition selon la revendication précédente, **caractérisée en ce que** la quantité de tensioactifs moussants anioniques, amphotères et zwitterioniques va de 0,5 à 15 % en poids par rapport au poids total de la composition.

16. Composition selon l'une quelconque des revendications précédentes, **caractérisée en ce qu'**elle contient en outre un dérivé anionique de protéines d'origine végétale ou de protéine de soie.

17. Composition selon l'une quelconque des revendications précédentes, **caractérisée en ce que** le sel d'ammonium quaternaire polymérique est choisi parmi les Polyquaternium.

18. Composition selon la revendication précédente, **caractérisée en ce que** le Polyquaternium est choisi parmi le Polyquaternium-7, le Polyquaternium-10, le Polyquaternium-39, le Polyquaternium-47 et leurs mélanges.

19. Composition selon l'une quelconque des revendications précédentes, **caractérisée en ce que** la quantité de sel(s) d'ammonium quaternaire polymérique va de 0,05 à 10 % en poids par rapport au poids total de la composition.

20. Composition selon l'une quelconque des revendications précédentes, **caractérisée en ce qu'**elle constitue un produit de nettoyage et/ou de démaquillage de la peau, un produit de douche, un shampoing, un après-shampoing, un produit de rasage, un masque à rincer, un produit exfoliant.

21. Utilisation cosmétique de la composition selon l'une quelconque des revendications 1 à 19, comme produit de nettoyage et/ou de démaquillage de la peau, comme produit de douche, comme shampoing, comme après-shampoing, comme produit de rasage, comme masque à rincer, comme produit exfoliant.

22. Procédé de nettoyage d'une matière kératinique, **caractérisé en ce qu'**on applique sur la matière kératinique, une composition selon l'une quelconque des revendications 1 à 19, et qu'on la rince.
